# DEMANDE DE BREVET EUROPEEN

(11) **EP 3 115 354 A1**
(43) Date de publication de la demande: **11.01.2017**
(21) Numéro de dépôt: 16178400.4
(22) Date de dépôt: 07.07.2016
(51) Int. Cl.: C07C 67/11, C11C 3/02

(54) **PROCÉDÉ DE PRODUCTION D'ESTER(S) DE POLYGLYCEROL**

(30) Priorité: 09.07.2015 FR 1556522
(71) Demandeur: Oleon N.V., 9940 Evergem (Ertvelde) (BE)
(72) Inventeur: CANIVENC, Edith, 60200 COMPIEGNE (FR)
(74) Mandataire: Santarelli

(57) **Abrégé**

La présente invention concerne un procédé de production d'ester(s) de polyglycérol ainsi que les esters de polyglycérols susceptibles d'être obtenus par ce procédé et leurs utilisations. Plus particulièrement, elle concerne un procédé de production d'un ester de polyglycérol comprenant la mise en contact, dans un réacteur, d'un acide gras et de carbonate de glycérol dans lequel le carbonate de glycérol est placé dans le réacteur, et au moins une partie de l'acide gras est ajouté dans le réacteur contenant le carbonate de glycérol chauffé à une température supérieure ou égale à 150°C.

## Description

La présente invention se rapporte à un procédé de production d'ester(s) de polyglycérol ainsi qu'aux esters de polyglycérols (PGE) susceptibles d'être obtenus par ce procédé et leurs utilisations.

L'un des défis importants de la chimie d'aujourd'hui est d'être plus respectueuse de l'environnement. Cette volonté d'être plus respectueuse de l'environnement est à l'origine de ce qui est communément appelé la « *chimie verte ».* Cette volonté se traduit à la fois dans les produits proposés et dans les procédés mis en oeuvre.

Concernant les produits, un des objectifs des industriels appliquant les principes de la chimie verte est de proposer au consommateur des produits plus sains (moins toxiques) et dont l'impact sur l'environnement est limité, par exemple en utilisant des réactifs biosourcés et/ou en proposant des produits biodégradables.

Concernant les procédés, les principes de la chimie verte se traduisent notamment par l'utilisation raisonnée des solvants, la limitation du nombre d'étapes dans une synthèse, l'économie d'énergie, par exemple en limitant les étapes de chauffage, leurs températures et/ou leurs durées, et la limitation des risques.

Depuis quelques années, diverses tentatives ont été faites afin de remplacer les tensioactifs d'origine pétrolière dont l'innocuité est sujette à question, tels que les tensioactifs basés sur des polyéthylèneglycols (PEG), par des produits plus respectueux de l'environnement, tels que les esters de polyglycérol.

Les esters de polyglycérols sont connus depuis le milieu du XXème siècle et ont notamment été utilisés en tant qu'émulsifiants dans les domaines cosmétiques et alimentaires. En effet, les esters de polyglycérol présentent une bonne innocuité et sont, de surcroît, biodégradables.

Un ester de polyglycérol est communément issu de la réaction d'estérification d'un polyglycérol et d'un acide gras. Le polyglycérol est lui-même issu de la réaction d'homopolymérisation du glycérol, aussi appelée réaction de polyaddition. Cette réaction d'homopolymérisation (ou de polyaddition) du glycérol consiste à faire réagir ensemble plusieurs molécules de glycérol via leurs fonctions hydroxyles résultant en la formation d'une molécule comportant plusieurs unités de glycérol liées par des liaisons éther.

Comme le glycérol, puis le polyglycérol, possèdent plusieurs fonctions hydroxyles, de nombreux produits sont susceptibles de se former. A titre d'exemple, la réaction de deux molécules de glycérol peut conduire à la formation des diglycérols suivants :

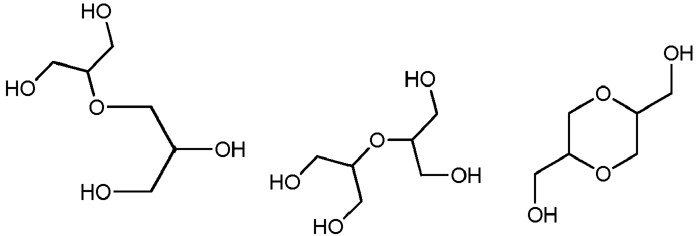

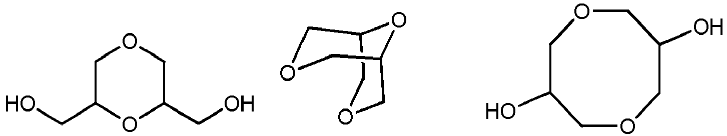

Par ailleurs, du fait de la présence de plusieurs fonctions hydroxyles sur le polyglycérol, celui-ci peut subir une ou plusieurs estérifications par un acide gras, conduisant à l'obtention de polyglycérols mono- ou poly-estérifiés. La réaction d'obtention de PGE conduit donc généralement à une composition comportant un mélange de PGE.

Cette composition d'esters de polyglycérol comporte donc plusieurs esters de polyglycérol qui différent les uns des autres par :
- le nombre d'unité(s) de glycérol formant le polyglycérol et l'agencement de ces unités,
- le degré d'estérification (c'est à dire le nombre d'acide(s) gras dans la molécule d'ester de polyglycérol), et éventuellement,
- la nature des acides gras dans la molécule d'ester de polyglycérol.

Alternativement, un ester de polyglycérol peut également être obtenu par réaction de carbonate de glycérol de formule : 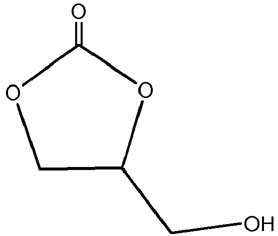 avec un acide gras.

La demande internationale WO 2008/142374 décrit un procédé de production d'un ester de polyglycérol comprenant la réaction d'un acide carboxylique d'un acide gras, avec du carbonate de glycérol. Selon WO 2008/142374, l'immiscibilité entre le carbonate de glycérol et l'acide carboxylique peut être évitée en utilisant des solvants appropriés tels que le diméthylisosorbide, le diméthylformamide, le diméthylsulfoxyde ou des dérivés éthérés. Cependant, dans la mesure du possible, il est souhaitable d'éviter l'utilisation de solvants organiques.

Actuellement, il existe toujours un besoin pour des procédés de production d'ester(s) de polyglycérol qui seraient respectueux des principes de la chimie verte, et permettraient l'obtention d'un haut rendement de production d'ester(s) de polyglycérol.

Le travail des inventeurs a permis de mettre en évidence qu'un procédé particulier de préparation d'ester(s) de polyglycérol permettait d'améliorer notablement le rendement de production en ester(s) de polyglycérol, tout en respectant au maximum les principes de la chimie verte.

L'invention concerne donc un procédé de production d'un ester de polyglycérol comprenant la mise en contact, dans un réacteur, d'un acide gras et de carbonate de glycérol dans lequel le carbonate de glycérol est placé dans le réacteur, et au moins une partie de l'acide gras est ajouté dans le réacteur contenant le carbonate de glycérol chauffé à une température supérieure ou égale à 150°C.

Selon le procédé de production de l'invention, les esters de polyglycérol sont obtenus par estérification du groupe carboxylique d'un acide gras sur une fonction hydroxyle d'un polyglycérol ou d'un carbonate de glycérol, ledit carbonate de glycérol subissant l'addition d'autres carbonates de glycérol subséquente. Lorsque l'estérification s'effectue sur une fonction hydroxyle en provenance d'un carbonate de glycérol, celle-ci se produit avant ou après ouverture du cycle au niveau de la fonction carbonate et en présence de dégagement de CO₂.

Deux mécanismes de formation de l'ester de polyglycérol selon le procédé selon l'invention sont particulièrement envisagés.

Lorsque l'acide gras réagit avec un carbonate de glycérol, il se forme un ester de glycérol. L'ester de polyglycérol est ensuite obtenu par réaction de polyaddition de carbonate de glycérol sur l'ester de glycérol.

Alternativement, l'acide gras réagit avec un polyglycérol préalablement formé par réaction d'homopolymérisation du carbonate de glycérol.

L'acide gras mis en contact avec le carbonate de glycérol est un acide gras comportant une chaine carbonée comportant au moins 8 atomes de carbone, de préférence au moins 10 atomes de carbone, de préférence encore au moins 12 atomes de carbone, de manière plus préférée au moins 16 atomes de carbone, et de manière encore plus préférée au moins 18 atomes de carbone. L'acide gras peut être saturé ou insaturé et/ou comporter une chaine carbonée linéaire ou ramifiée.

De préférence, l'acide gras est un acide gras saturé de formule brute CₙH₂ₙO₂ dans laquelle n est un entier, de préférence pair, compris entre 8 et 24, de préférence entre 14 et 20.

Un acide gras particulièrement préféré pour la mise en oeuvre du procédé de l'invention est l'acide isostéarique (Iso C18:0).

On notera que dans le cadre de la présente demande, et sauf stipulation contraire, les gammes de valeurs indiquées s'entendent bornes incluses.

Selon le procédé de l'invention, il est également possible de mettre en contact au moins deux acides gras de nature chimique différente (c'est-à-dire ne comportant pas une chaine carbonée identique) avec le carbonate de glycérol, placé dans le réacteur. Dans ce cas, les acides gras pourront être introduits dans le réacteur séparément ou sous la forme d'un mélange préalablement préparé. De préférence, chaque acide gras mis en contact avec le carbonate de glycérol comporte une chaine carbonée comportant au moins 8 atomes de carbone, de préférence au moins 10 atomes de carbone, de préférence encore au moins 12 atomes de carbone, de manière plus préférée au moins 16 atomes de carbone, et de manière encore plus préférée au moins 18 atomes de carbone. Chaque acide gras peut être saturé ou insaturé et/ou comporter une chaine carbonée linéaire ou ramifiée. De préférence, chaque acide gras est un acide gras saturé de formule brute CₙH₂ₙO₂ dans laquelle n est un entier, de préférence pair, compris entre 8 et 24, de préférence entre 14 et 20. De préférence, au moins un des acides gras mis en contact avec le carbonate de glycérol est l'acide isostéarique (Iso C18:0).

Au moins une partie de l'acide gras est ajouté dans le réacteur contenant le carbonate de glycérol chauffé à une température supérieure ou égale à 150°C. De préférence, le carbonate de glycérol est chauffé à une température supérieure ou égale à 170°C. Par « chauffé à une température », on entend que le réactif est chauffé et a atteint cette température.

Plus préférentiellement encore, le carbonate de glycérol est chauffé à une température appelée « température de réaction ». La température de réaction du procédé de production d'ester(s) de polyglycérol selon l'invention est celle, usuelle, d'une réaction d'estérification. Cette température de réaction est en particulier celle à laquelle on chauffe le milieu réactionnel, une fois l'acide gras introduit en totalité dans le réacteur.

La température de réaction est avantageusement comprise entre 170°C et 250°C. De préférence, cette température de réaction est comprise entre 180°C et 230°C, plus préférentiellement entre 200°C et 220°C. De manière encore plus préférée, la température de réaction est de 210°C.

A titre indicatif, la température de réaction peut varier selon la longueur de la chaine carbonée de l'acide gras mis en contact avec le carbonate de glycérol. En effet, de manière générale, plus la chaîne carbonée est longue, plus la température sera avantageusement élevée.

Plus particulièrement, lorsqu'il est indiqué que « l'acide gras est ajouté dans le réacteur contenant le carbonate de glycérol chauffé », cela signifie que ledit carbonate de glycérol n'a pas totalement réagit par polymérisation pour donner du polyglycérol et que des molécules de carbonate de glycérol sont encore présentes dans le réacteur.

Avantageusement, le début de l'ajout d'au moins une partie de l'acide gras dans le réacteur est effectué dans un délai compris entre le moment où le carbonate de glycérol atteint une température de 150°C et jusqu'à 2h00 après que cette température soit atteinte. De préférence, le début de l'ajout d'au moins une partie de l'acide gras est effectué dans un délai compris entre le moment où le carbonate de glycérol atteint la température de 150°C et jusqu'à 1h30, préférentiellement 1h00, plus préférentiellement 30 min après que cette température soit atteinte, encore plus préférentiellement dès que cette température est atteinte.

Généralement, sur un réacteur industriel, une augmentation de la température de 150°C à 210°C est réalisée sur une durée allant jusqu'à 2h00. A l'échelle du laboratoire, une telle augmentation de température peut être obtenue en moins de 30 min.

Ce délai de 2h00 suivant l'obtention de la température de 150°C permet d'éviter que la réaction d'homopolymérisation du carbonate de glycérol ne soit complète, c'est-à-dire d'éviter que la totalité du carbonate de glycérol ne se soit transformé en polyglycérol et qu'il ne reste plus de carbonate de glycérol disponible dans le réacteur. L'achèvement de cette réaction d'homopolymérisation du carbonate de glycérol nécessite a minima une durée de 3h suivant l'initiation de la réaction, l'initiation de la réaction d'homopolymérisation ayant lieu généralement lorsque la température du carbonate de glycérol atteint la température de 150°C. D'autres facteurs peuvent également influencer cette initiation tels que la présence éventuelle d'un catalyseur.

En effet, il est avantageux qu'au moins une partie de l'acide gras ajouté dans le réacteur, puisse réagir avec un carbonate de glycérol. De préférence, au moins une partie de l'acide gras est ajouté dans le réacteur contenant du carbonate de glycérol chauffé, le carbonate de glycérol correspondant à au moins 5% en poids sur le poids total de réactif(s) glycérol.

Par « poids total de réactif(s) glycérol », on vise le poids total de carbonate de glycérol et éventuellement de polyglycérol pouvant être formé.

Préférentiellement, le carbonate de glycérol correspond à au moins 10% en poids sur le poids total de réactif(s) glycérol, plus préférentiellement au moins 30% en poids sur le poids total de réactif(s) glycérol, encore plus préférentiellement au moins 50% en poids sur le poids total de réactif(s) glycérol.

L'homme du métier sait comment démontrer que la réaction d'homopolymérisation du carbonate de glycérol n'est pas achevée par exemple en détectant par infra-rouge la présence du signal pour la liaison C=O du carbonate de glycérol.

Le procédé de l'invention conduit généralement à l'obtention de deux phases en fin de synthèse, une phase contenant du polyglycérol (issu de l'homopolymérisation de carbonate de glycérol qui n'a pas réagi avec l'acide gras), appelée « phase PG », et une phase contenant les esters de polyglycérol, appelée « phase PGE ». Ces phases peuvent être séparées par des méthodes de séparation bien connues de l'art antérieur, telle que la décantation, de préférence la décantation à chaud. Avantageusement, la décantation s'effectue à une température comprise entre 50°C et 120°C, préférentiellement entre 70°C et 100°C.

L'ajout d'au moins une partie de l'acide gras dans le réacteur contenant le carbonate de glycérol chauffé permet l'obtention d'une phase à haut rendement en esters de polyglycérol en fin de synthèse, c'est-à-dire que la quantité d'ester(s) de polyglycérol obtenue en fin de synthèse est bien supérieure à la quantité de polyglycérol (PG) (voir en particulier les tableaux 1 et 2a). Notamment, un rapport massique PGE/PG supérieur à 80/20 est obtenu pour un ratio molaire carbonate de glycérol/acide gras de 4/1.

De manière similaire, le délai entre le moment où le carbonate de glycérol atteint la température de 150°C et l'ajout d'au moins une partie de l'acide gras permet avantageusement de contrôler la répartition de l'obtention de ces deux phases en favorisant l'obtention de la phase contenant les esters de polyglycérol.

L'ajout d'au moins une partie de l'acide gras dans le réacteur contenant le carbonate de glycérol chauffé est également nécessaire pour obtenir un PGE dispersable à 1% en poids dans de l'eau déminéralisée, la dispersion en résultant étant stable sur une période d'au moins 5 jours, à température ambiante.

De préférence, le réacteur utilisé pour la mise en oeuvre du procédé selon l'invention est un réacteur agité et semi-fermé. Par réacteur agité, on entend tout type de réacteur dont les réactifs qu'il contient sont soumis à une agitation, généralement par l'intermédiaire d'un système mécanique, tel que par exemple à l'aide d'un agitateur mécanique. L'utilisation d'un réacteur semi-fermé est particulièrement avantageuse. En effet, la réaction d'estérification s'effectue préférentiellement sous atmosphère inerte, telle que sous azote.

Avantageusement, le réacteur est muni d'un système de distillation afin d'éliminer l'eau formée durant la réaction d'estérification.

Selon un premier mode de réalisation du procédé de production, la quantité totale d'acide gras est ajoutée dans le réacteur en une fois. Toutefois, au moins une partie de l'acide gras est ajoutée dans le réacteur contenant le carbonate de glycérol chauffé à une température supérieure ou égale à 150°C.

Par « quantité totale d'acide gras », on désigne la quantité d'acide gras introduite dans le réacteur au cours du procédé de production selon l'invention.

Par conséquent, deux modes particuliers de ce premier mode de réalisation peuvent être proposés :
a) l'acide gras est introduit dans le réacteur selon un écoulement continu, une partie dudit écoulement étant réalisé alors que le carbonate de glycérol est chauffé à une température supérieure ou égale à 150°C, ou
b) la quantité totale d'acide gras est introduite selon un écoulement continu dans le réacteur contenant le carbonate de glycérol chauffé à une température supérieure ou égale à 150°C.

Par « écoulement continu », on désigne un écoulement sans interruption. Un tel écoulement peut être obtenu, à l'échelle du laboratoire, par une ampoule de coulée ou une pompe péristaltique. A l'échelle industrielle, un écoulement continu peut être également obtenu par une pompe péristaltique ou une pompe volumétrique.

A titre d'exemple du mode particulier a), l'écoulement continu peut être initié alors que le carbonate de glycérol est placé dans le réacteur, sans que le carbonate de glycérol ne soit chauffé ou alors que le carbonate de glycérol est chauffé à une température inférieure à 150°C, et se poursuivre durant le chauffage du carbonate de glycérol jusqu'à ce que ce dernier atteigne la température souhaitée, par exemple au moins 150°C ou une température de réaction. De préférence, l'écoulement se poursuit après que le carbonate de glycérol ait atteint la température souhaitée.

A titre d'exemple du mode particulier b), le carbonate de glycérol est placé dans le réacteur puis chauffé. Avantageusement, lorsque le carbonate de glycérol atteint la température souhaitée, à savoir au moins 150°C ou une température de réaction, l'écoulement continu est initié.

Dans les deux modes particuliers ci-avant, l'écoulement peut également se poursuivre alors que le carbonate de glycérol est porté à une seconde température. De préférence, la seconde température est d'au moins 150°C ou une seconde température de réaction.

Selon un second mode de réalisation du procédé de production, la quantité totale d'acide gras est ajoutée dans le réacteur en au moins deux fois. Toutefois, au moins une partie de l'acide gras est ajoutée dans le réacteur contenant le carbonate de glycérol chauffé à une température supérieure ou égale à 150°C.

Par conséquent, deux modes particuliers de ce second mode de réalisation peuvent être proposés :
c) une partie de l'acide gras est introduite avant que le carbonate de glycérol ne soit chauffé à une température supérieure ou égale à 150°C, une autre partie de l'acide gras étant introduite une fois le carbonate de glycérol chauffé à une température supérieure ou égale à 150°C,
d) la totalité de l'acide gras est introduite en au moins deux fois, après que le carbonate de glycérol ait été chauffé à une température supérieure ou égale à 150°C.

De préférence, l'acide gras est ajouté en au moins 3 fois dans le réacteur contenant le carbonate de glycérol. On notera que lorsque l'acide gras est ajouté en plusieurs fois ou parties, la somme des quantités ajoutées dans le réacteur au cours du procédé de production équivaut à la quantité totale d'acide gras.

A titre d'exemple du mode particulier c), le carbonate de glycérol est placé dans le réacteur avec une partie de l'acide gras, préalablement au chauffage. Alternativement, une partie de l'acide gras est introduite dans le réacteur alors que le carbonate de glycérol est chauffé à une température inférieure à 150°C. Puis le carbonate de glycérol et l'acide gras présent dans le réacteur sont chauffés de manière à ce que le carbonate de glycérol ait atteint la température souhaitée, par exemple au moins 150°C ou une température de réaction. Lorsque le carbonate de glycérol est chauffé à la température souhaitée, une autre partie de l'acide gras est introduite dans le réacteur. Si l'acide gras est ajouté en au moins trois fois, il est possible de faire varier la température du carbonate de glycérol, par exemple, afin de porter le carbonate de glycérol à une seconde température. De préférence, la seconde température est d'au moins 150°C ou une seconde température de réaction. Une autre partie de l'acide gras peut alors être introduite dans le réacteur.

A titre d'exemple du mode particulier d), le carbonate de glycérol est placé dans le réacteur puis chauffé à une température souhaitée, à savoir une température supérieure ou égale à 150°C ou une température de réaction. Avantageusement, lorsque le carbonate de glycérol atteint la température souhaitée, une première partie de l'acide gras est introduite dans le réacteur. Une deuxième partie de l'acide gras peut être introduite après l'ajout de la première partie, les deux ajouts étant séparés par un intervalle de temps donné. Il est possible durant cet intervalle de temps de faire varier la température du carbonate de glycérol, par exemple, afin de porter le carbonate de glycérol à une seconde température. De préférence, la seconde température est d'au moins 150°C ou une seconde température de réaction.

Par « intervalle de temps », on désigne le temps écoulé entre la fin d'un ajout d'acide gras et le début de l'ajout suivant d'acide gras.

Selon un mode préféré du second mode de réalisation, au moins une partie de l'acide gras est ajouté goutte à goutte dans le réacteur contenant le carbonate de glycérol. Par « goutte à goutte », on entend les ajouts répétés d'une quantité donnée (ou partie) d'un réactif, à savoir l'acide gras, les ajouts étant séparés par un intervalle de temps donné. De préférence, la quantité totale d'acide gras est ajoutée goutte à goutte dans le réacteur.

Le goutte à goutte peut être réalisé avantageusement à l'aide d'une pompe péristaltique, qui permet notamment de contrôler la quantité d'acide gras introduite dans le réacteur lors de chaque ajout et l'intervalle de temps entre chaque ajout. Le goutte à goutte peut également être réalisé au moyen d'une ampoule de coulée.

La quantité d'acide gras introduite lors de chaque ajout, la durée de chaque ajout et l'intervalle de temps entre chaque ajout peuvent être identiques ou différents.

De préférence et quel que soit le mode de réalisation, la quantité totale d'acide gras est ajoutée dans le réacteur sur une période de temps d'au moins 30 min, c'est-à-dire que :
- dans le cas où l'acide gras est ajouté en une seule fois, la période de temps écoulée entre le début de l'ajout et la fin de l'ajout de l'acide gras est d'au moins 30 min,
- dans le cas où l'acide gras est ajouté en au moins deux fois, la période de temps écoulée entre le début de l'ajout de la première partie de l'acide gras et la fin de l'ajout de la dernière partie de l'acide gras est d'au moins 30 min.

Avantageusement, la quantité totale d'acide gras est ajoutée dans le réacteur sur une période de temps comprise entre 1 et 12h. De préférence, l'ajout est réalisé sur une période de temps comprise entre 2 et 10h. De manière encore plus préférée, l'ajout est réalisé sur une période de temps comprise entre 3 et 7h.

A titre d'exemple, l'ajout pendant une période de temps donnée de l'acide gras dans le réacteur contenant le carbonate de glycérol peut correspondre à un débit massique moyen d'acide gras de l'ordre de 25 à 40% de la quantité totale d'acide gras par heure, préférentiellement de l'ordre de 30 à 35%. Le débit massique est qualifié de « moyen » car l'introduction de l'acide gras pouvant s'effectuer selon un écoulement continu ou par au moins deux ajouts successifs, le débit est calculé par la moyenne de la quantité introduite dans le réacteur sur une période d'une heure.

Selon les conditions de l'ajout de l'acide gras, le rapport massique PGE/PG obtenu en fin de synthèse est supérieur à celui obtenu par un procédé en masse. Par « procédé en masse », on entend un procédé de production d'un ester de polyglycérol comprenant la mise en contact, dans un réacteur, de la quantité totale d'acide gras et de carbonate de glycérol avant chauffage à une température de réaction. Selon les conditions de l'ajout de l'acide gras, en fin de synthèse, il est possible d'obtenir un rapport massique PGE/PG supérieur 80/20, de préférence supérieur à 90/10, et de manière encore plus préférée supérieur à 95/5.

Avantageusement, dans le procédé de l'invention, il est possible d'utiliser un catalyseur basique.

Le catalyseur basique peut être homogène, c'est-à-dire qu'il ne forme qu'une phase avec au moins l'un des réactifs (carbonate de glycérol et/ou éventuellement l'acide gras) avec lesquels il est mis en contact, et/ou hétérogène. En particulier, il est possible d'introduire plusieurs catalyseurs basiques dans le réacteur.

Des exemples de catalyseurs basiques appropriés sont des hydroxydes de métal alcalin, tels que NaOH, KOH, LiOH, Ca(OH)₂ ou CaO, des carbonates ou bicarbonates de métal alcalin, tels que K₂CO₃, Na₂CO₃, KHCO₃, NaHCO₃ ou CaCO₃ des alkoxydes de métal alcalin ou des amines tertiaires.

Avantageusement, le catalyseur basique est introduit dans le réacteur à une teneur de 0,1 à 10 %, préférentiellement 0,1 à 8 % molaire par rapport à la quantité totale d'acide gras introduite ou qui sera introduite dans le réacteur.

L'ajout du catalyseur basique à une teneur allant de 0,1 à 8% molaire par rapport à la quantité totale d'acide gras a pour avantage d'éviter ou de limiter la coloration de la phase PGE.

Les catalyseurs basiques particulièrement appropriés sont des catalyseurs permettant d'obtenir un ester de polyglycérol ayant des caractéristiques organoleptiques favorables à une utilisation cosmétique, telles qu'une odeur et une couleur de basse intensité. Un tel catalyseur est par exemple l'hydroxyde de calcium (Ca(OH)₂), également appelé chaux éteinte.

Avantageusement, le catalyseur basique est introduit dans le réacteur, préalablement à tout ajout d'acide gras ou alternativement, avant que le carbonate de glycérol n'ait atteint la température de 170°C.

De préférence, un agent compatibilisant est également introduit dans le réacteur.

Par « agent compatibilisant », on vise un agent qui facilite en effet la solubilisation des réactifs entre eux, et notamment de l'acide gras mis en contact avec le carbonate de glycérol, en diminuant la tension interfaciale entre ces réactifs. La tension interfaciale entre le carbonate de glycérol, le polyglycérol et l'acide gras est d'autant plus importante que la longueur de la chaine carbonée (c'est-à-dire le nombre d'atomes de carbone présents dans la chaîne carbonée) de l'acide gras est élevée. Il est donc particulièrement avantageux d'utiliser un agent compatibilisant lorsque l'acide gras mis en contact avec le carbonate de glycérol comporte une chaine carbonée comportant au moins 14 atomes de carbone, de sorte à diminuer la tension interfaciale pouvant exister entre ces réactifs.

Avantageusement, l'agent compatibilisant introduit dans le réacteur est un ester de polyglycérol. L'utilisation d'un tel agent compatibilisant permet d'éviter l'emploi de solvant(s) et par conséquent, de limiter l'impact du procédé de production selon l'invention sur l'environnement. Par conséquent, le procédé selon l'invention peut être réalisé sans solvant.

Plus particulièrement, l'agent compatibilisant est un ester de polyglycérol issu de la réaction entre du carbonate de glycérol et un acide gras. De préférence, l'agent compatibilisant est un ester de polyglycérol ou un mélange d'esters de polyglycérol susceptible d'être obtenu selon le procédé de production selon l'invention.

Avantageusement, l'ester de polyglycérol utilisé comme agent compatibilisant a une chaine carbonée comportant un nombre de carbones approximativement identique au nombre de carbones de la chaine carbonée d'au moins un acide gras mis en réaction dans le procédé de production de PGE selon l'invention. Par approximativement, on entend que le nombre de carbones est identique à +/- 4 carbones, préférentiellement identique à +/- 2 carbones, plus préférentiellement identique.

L'ajout d'un agent compatibilisant tel qu'un PGE permet également d'améliorer certaines propriétés des esters de polyglycérols obtenus par le procédé selon l'invention.

En effet, de manière surprenante, l'ajout de PGE en tant qu'agent compatibilisant a pour avantage de diminuer la présence de microgels dans les phases contenant les produits obtenus à l'issue du procédé selon l'invention. Les microgels pouvant être présents dans les phases PG et PGE en fin de synthèse, proviennent de réactions secondaires qui ont lieu pendant le procédé de production d'ester(s) de polyglycérol de l'invention. Ces réactions secondaires sont, par exemple, des réactions d'éthérification entre des fonctions hydroxyles PG, qui conduisent à la formation de réticulations et *in fine,* de microgels. La faible présence de microgels, dans les phases PG et PGE en fin de synthèse, est donc un indicateur de la qualité et de l'efficacité du procédé mis en oeuvre. En outre, les microgels peuvent avoir un impact sur la viscosité des phases PG et PGE obtenues.

De plus, l'ajout d'agent compatibilisant permet de diminuer la quantité de catalyseur sans nuire au rendement en PGE.

L'ajout de PGE en tant qu'agent compatibilisant a également pour avantage de diminuer l'indice d'acide de la phase PGE obtenue en fin de synthèse, pour un temps de réaction donné. L'indice d'acide représente la quantité d'acides gras libres dans un corps gras. Il correspond à la masse d'hydroxyde de potassium (potasse) en milligrammes, fixée à froid par gramme de corps gras. Plus l'indice d'acide est faible dans la phase PGE, moins la quantité d'acides gras libres est importante. Un faible indice d'acide, de préférence inférieur à 2, est donc un indicateur du degré d'avancement de la réaction mise en oeuvre dans le procédé et *in fine* de son achèvement.

Enfin, lorsque le ratio de carbonate de glycérol sur l'acide gras est de 4/1, l'ajout de PGE en tant qu'agent compatibilisant permet l'obtention, en fin de synthèse, d'une phase PGE dispersable à 1 % en poids dans de l'eau déminéralisée après agitation à température ambiante.

Par « dispersable », on entend une dispersion qui, stockée à température ambiante (c'est-à-dire 20°C dans les conditions normales de température et de pression, CNTP), est stable dans le temps (pas de dépôt visible à l'oeil nu après au moins cinq jours de stockage). Un exemple de préparation de ces dispersions est décrit à l'Exemple 4 ci-après. Les esters de polyglycérol obtenus peuvent donc facilement être mis en oeuvre dans des préparations, telles que des émulsions, en particulier des préparations cosmétiques.

Le poids d'agent compatibilisant, et plus particulièrement de PGE, qui est introduit dans le réacteur par rapport au poids total de la masse réactionnelle est de 0,1 à 10%. Par « poids total de la masse réactionnelle », on vise le poids total de l'ensemble des réactifs et éventuellement du catalyseur mis en oeuvre dans le procédé de production d'ester(s) de polyglycérol selon l'invention. De préférence, le poids d'agent compatibilisant qui est introduit dans le réacteur par rapport au poids total de la masse réactionnelle est de 0,5 à 5%. De manière encore plus préférée, le poids d'agent compatibilisant qui est introduit dans le réacteur par rapport au poids total de la masse réactionnelle est de 0,8 à 2 %.

Avantageusement, l'agent compatibilisant est introduit dans le réacteur, préalablement à tout ajout d'acide gras.

Selon un mode de réalisation préférentiel du procédé de production selon l'invention, le catalyseur basique introduit dans le réacteur contenant le carbonate de glycérol est plus préférentiellement l'hydroxyde de calcium (Ca(OH)₂). Ce catalyseur permet d'obtenir une phase PGE ayant des caractéristiques organoleptiques favorables telles qu'odeur et couleur de basse intensité. De telles caractéristiques sont particulièrement appropriées pour une utilisation cosmétique. Plus particulièrement, ce catalyseur permet l'obtention d'une phase PGE présentant une couleur ayant un indice inférieur ou égal à 6 sur l'échelle de Gardner. L'indice de Gardner est un indice de couleur pour les liquides transparents bien connu de l'homme du métier. Sa mesure est décrite dans la norme ASTM D1544-98.

De préférence, l'hydroxyde de calcium est introduit à une quantité de 0,1 à 7,5% molaire par rapport à la quantité totale d'acide gras introduite. De préférence, la quantité d'hydroxyde de calcium introduite est de 0,2 à 6% molaire par rapport à la quantité totale d'acide gras introduite. De manière encore plus préférée, la quantité d'hydroxyde de calcium introduite est de 0,3 à 5,5% molaire par rapport à la quantité totale d'acide gras introduite.

Selon un mode particulier de réalisation du mode de réalisation préférentiel, la quantité d'hydroxyde de calcium introduite est de 0,3 à 1% molaire par rapport à la quantité totale d'acide gras introduite. Une faible teneur en catalyseur présente l'avantage de diminuer la présence de microgels dans les phases PGE et PG obtenues en fin de synthèse. En outre, ce mode particulier de réalisation du mode de réalisation préférentiel, lorsqu'il est combiné avec l'introduction d'un agent compatibilisant, permet d'augmenter le rapport massique PGE/PG obtenu par le procédé selon l'invention.

Lorsque le procédé de l'invention met en oeuvre un catalyseur basique et/ou un agent compatibilisant, il est possible de chauffer le carbonate de glycérol présent dans le réacteur avant l'ajout du catalyseur basique et/ou de l'agent compatibilisant. En effet, l'effet du chauffage combiné à l'ajout de l'agent compatibilisant et plus particulièrement à l'ajout du catalyseur dans le réacteur contenant le carbonate de glycérol va favoriser la réaction d'homopolymérisation du carbonate de glycérol.

Par conséquent, si l'on souhaite limiter le nombre d'unité de glycérol dans le polyglycérol de l'ester de polyglycérol, il sera nécessaire de contrôler la réaction d'homopolymérisation du carbonate de glycérol durant la montée en température du réacteur jusqu'à une température de réaction. Pour ce faire, il peut être avantageux de chauffer le carbonate de glycérol à une température d'introduction comprise entre 130°C et 190°C, de préférence entre 140°C et 180°C, de manière encore plus préférée entre 150°C et 170°C, avant l'introduction de l'agent compatibilisant et/ou du catalyseur.

Une fois que le carbonate de glycérol a atteint la température d'introduction, l'agent compatibilisant et/ou le catalyseur sont introduits dans le réacteur. Le milieu réactionnel ainsi formé est ensuite à nouveau chauffé pour atteindre une température souhaitée, à savoir une température d'au moins 150°C ou une température de réaction.

Une fois la température souhaitée atteinte, l'acide gras peut être introduit dans le réacteur selon le premier ou le second mode de réalisation décrit ci-avant. Avantageusement, le début de l'ajout d'au moins une partie de l'acide gras est effectué dans les 2h00, préférentiellement dans les 1h30 suivant l'atteinte de la température de 150°C par le carbonate de glycérol, plus préférentiellement dans l'heure, encore plus préférentiellement dans les 30 min, par exemple dès que le carbonate de glycérol atteint la température de 150°C.

Lorsque l'acide gras a été introduit dans le réacteur, le carbonate de glycérol et l'acide gras sont chauffés à une température de réaction, comprise entre 170°C et 250°C. Plus préférentiellement, cette température de réaction est maintenue lorsque la quantité totale d'acide gras a été introduite dans le réacteur.

La réaction d'estérification s'effectue, de préférence, en milieu inerte. Par exemple, le milieu inerte est induit par bullage en continu d'azote dans le milieu réactionnel.

Avantageusement dans les procédés de l'invention, le ratio molaire de carbonate de glycérol/acide gras qui sont introduits dans le réacteur, est supérieur ou égal à 2.

Par « ratio molaire de carbonate de glycérol/acide gras », on vise le ratio de la quantité de carbonate de glycérol placé dans le réacteur sur la quantité totale d'acide gras introduit dans le réacteur.

De préférence, le ratio molaire de carbonate de glycérol/acide gras qui sont introduits dans le réacteur, est supérieur ou égal à 3. De manière encore plus préférée, le ratio molaire de carbonate de glycérol/acide gras qui sont introduits dans le réacteur, est supérieur ou égal à 4, tel que par exemple, un ratio de 4 ou 6.

De manière surprenante, il est possible en effet d'obtenir un PGE dispersable et stable avec tous ces ratios (4/1, 6/1, 10/1) à l'aide d'un procédé selon l'invention.

Un premier procédé de production de PGE selon l'invention particulièrement préféré comporte les étapes suivantes :
(i) introduction du carbonate de glycérol dans un réacteur,
(ii) chauffage du carbonate de glycérol à une température comprise entre 150°C à 170°C, de préférence environ 160°C,
(iii) introduction de l'agent compatibilisant, de préférence un PGE, et du catalyseur, de préférence du Ca(OH)₂,
(iv) chauffage du milieu réactionnel ainsi obtenu à une température comprise entre 180°C et 220°C, de préférence d'environ 210°C,
(v) ajout goutte à goutte ou en écoulement continu de l'acide gras sur une période de temps d'au moins 1h30, préférentiellement au moins 2h30, le ratio molaire de carbonate de glycérol / acide étant supérieur à 2, de préférence de 4,
(vi) maintien du chauffage du milieu réactionnel ainsi obtenu à une température comprise entre 180°C et 220°C, de préférence à environ 210°C, jusqu'à l'obtention d'un indice d'acide inférieur ou égal à 2,5, préférentiellement inférieur ou égal à 2.

Avantageusement, l'étape (v) de ce premier procédé de production de PGE débute dans les 2h00, préférentiellement dans les 1h30, plus préférentiellement dans l'heure après que le carbonate de glycérol ait atteint la température de 150°C. Au laboratoire, ce délai peut être notablement diminué car les temps de chauffage sont diminués. Ainsi, l'étape (v) de ce premier procédé de production de PGE peut débuter dans les 15 à 30 min après que le carbonate de glycérol ait atteint la température de 150°C.

Un second procédé de production de PGE selon l'invention particulièrement préféré comporte les étapes suivantes :
(i) introduction du carbonate de glycérol et du catalyseur, de préférence du Ca(OH)₂, dans un réacteur,
(ii) chauffage du milieu réactionnel ainsi obtenu à une température comprise entre 180°C et 220°C, de préférence d'environ 210°C,
(iii) ajout goutte à goutte ou en écoulement continu de l'acide gras sur une période de temps d'au moins 1h30, préférentiellement au moins 2h30, le ratio molaire de carbonate de glycérol / acide étant supérieur à 2, de préférence de 6,
(iv) maintien du chauffage du milieu réactionnel ainsi obtenu à une température comprise entre 180°C et 220°C, de préférence à environ 210°C, jusqu'à l'obtention d'un indice d'acide inférieur ou égal à 2,5, préférentiellement inférieur ou égal à 2.

Avantageusement, l'étape (iii) de ce deuxième procédé de production de PGE débute dans les 2h00, préférentiellement dans les 1h30, plus préférentiellement dans l'heure après que le carbonate de glycérol ait atteint la température de 150°C. Au laboratoire, ce délai peut être notablement diminué car les temps de chauffage sont diminués. Ainsi, l'étape (iii) de ce second procédé de production de PGE peut débuter dans les 15 à 30 min après que le carbonate de glycérol ait atteint la température de 150°C.

Un troisième procédé de production de PGE selon l'invention particulièrement préféré comporte les étapes suivantes :
(i) introduction du carbonate de glycérol dans un réacteur,
(ii) chauffage du carbonate de glycérol à une température comprise entre 150°C à 170°C, de préférence environ 160°C,
(iii) introduction de l'agent compatibilisant, de préférence un PGE, et du catalyseur, de préférence du Ca(OH)₂,
(iv) chauffage du milieu réactionnel ainsi obtenu à une température comprise entre 180°C et 220°C, de préférence d'environ 210°C,
(v) ajout goutte à goutte ou en écoulement continu de l'acide gras sur une période de temps d'au moins 1h30, préférentiellement au moins 2h30, le ratio molaire de carbonate de glycérol / acide étant supérieur à 2, de préférence de 6,
(vi) maintien du chauffage du milieu réactionnel ainsi obtenu à une température comprise entre 180°C et 220°C, de préférence à environ 210°C, jusqu'à l'obtention d'un indice d'acide inférieur ou égal à 2,5, préférentiellement inférieur ou égal à 2.

Avantageusement, l'étape (v) de ce troisième procédé de production de PGE débute dans les 2h00, préférentiellement dans les 1h30, plus préférentiellement dans l'heure après que le carbonate de glycérol ait atteint la température de 150°C. Au laboratoire, ce délai peut être notablement diminué car les temps de chauffage sont diminués. Ainsi, l'étape (v) de ce troisième procédé de production de PGE peut débuter dans les 15 à 30 min après que le carbonate de glycérol ait atteint la température de 150°C.

Plus particulièrement, les procédés selon l'invention particulièrement préférés présentent une, plusieurs ou toutes les caractéristique(s) préférée(s) mentionnée(s) dans les procédés de production décrits ci-avant. En particulier, l'acide gras est de préférence l'acide isostéarique. De plus :
- l'agent compatibilisant PGE est introduit à une teneur allant de 0,5 à 5% en poids par rapport au poids total de la masse réactionnelle, préférentiellement à une teneur de 0,8 à 2% en poids, et
- le catalyseur Ca(OH)₂ est introduit à une teneur allant de 0,3 à 5,5% en poids par rapport au poids total de la masse réactionnelle, préférentiellement à une teneur de 0,3 à 1% en poids.

Comme cela a été indiqué ci-avant, le procédé selon l'invention conduit à l'obtention d'un produit, qui après décantation, comporte une phase PG et une phase PGE. Le procédé selon l'invention comporte donc avantageusement une étape de décantation à l'issue de la réaction. Cette étape de décantation est avantageusement suivie d'une étape de séparation des phases afin notamment de récupérer le PGE. Une telle étape de séparation est bien connue de l'homme du métier.

L'invention concerne également les produits et notamment les esters de polyglycérol, susceptibles d'être obtenus par le procédé de production selon l'invention et plus particulièrement par les procédés selon l'invention particulièrement préférés.

En outre, l'invention concerne l'utilisation des produits susceptibles d'être obtenus par le procédé de l'invention, comme émulsifiant.

Les produits susceptibles d'être obtenus par le procédé de l'invention peuvent être utilisés comme agent émulsifiant dans le domaine de l'alimentaire ou de la cosmétique. Ils peuvent en particulier être utilisés pour produire, stabiliser et disperser des émulsions. Les produits selon l'invention peuvent être mis en oeuvre dans la préparation d'émulsions eau dans huile ou huile dans eau ainsi que dans la préparation d'émulsions multiples (par exemple : eau/huile/eau).

De préférence, les produits selon l'invention seront utilisés comme émulsifiant dans des compositions cosmétiques telles que des crèmes à application cutanée, des crèmes pour nourrissons, des crèmes hydratantes ou encore des crèmes protectrices.

Les produits selon l'invention peuvent également être utilisés en tant qu'épaississant, inhibiteur de cristallisation dans le domaine cosmétique ou le domaine alimentaire.

L'invention sera mieux comprise au vu des exemples qui suivent, donnés à titre illustratif, avec référence à :

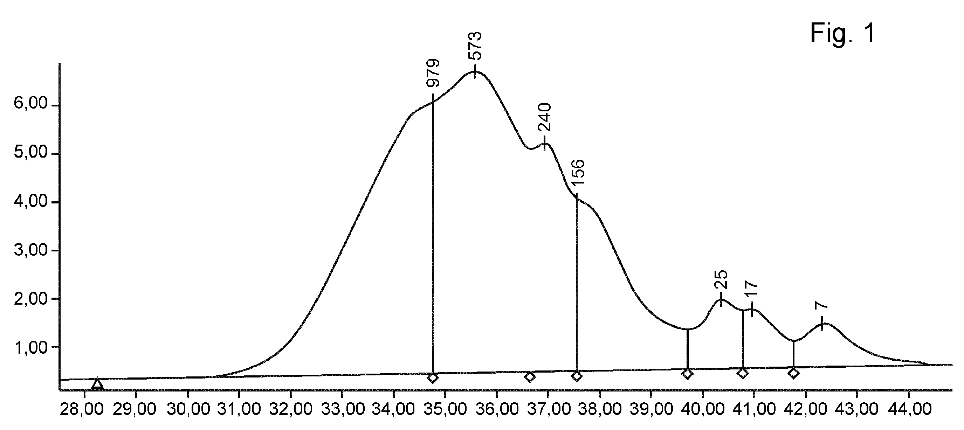

- La Figure 1 qui représente le chromatogramme d'exclusion stérique du produit obtenu dans l'essai 8 de l'exemple 4.

D'autres caractéristiques et avantages de l'invention, apparaîtront dans les exemples qui suivent, donnés à titre illustratif.

### Exemple 1 : Procédé de production d'ester(s) de polyglycérol selon l'invention

### Essai 1

Dans un réacteur en verre de 500 ml équipé d'une agitation mécanique, d'une sortie directe de distillation et d'une introduction d'azote dans le milieu réactionnel, on charge 495,6 g (4,20 mole) de carbonate de glycérol et 3,9 g (0,05 mole) d'hydroxyde de calcium (chaux). Le milieu réactionnel est chauffé progressivement à 200°C, puis 298,5 g (1,05 mole) d'acide isostéarique (désigné ci- après « Iso C18:0 ») Radiacid® 909 de la société OLEON sont additionnés goutte à goutte au moyen d'une pompe péristaltique pendant 3 heures. Un prélèvement est réalisé pour titrage de l'indice d'acide en fin d'introduction puis le milieu réactionnel est maintenu pendant 3 heures à 210°C. L'évolution de la réaction est suivie par titrage d'indice d'acide sur prélèvements. Après refroidissement, on obtient 544,7 g de masse réactionnelle dont 501,1 g d'ester de polyglycérol (PGE) d'indice d'acide: 2,3 mg KOH/g en phase supérieure et 43,6 g de polyglycérol (PG) en phase inférieure contenant des microgels, après décantation ; soit un ratio PGE/PG de 92/8. 21,4 g d'eau distillée sont récupérés.

La présence de microgels est estimée de manière qualitative au moyen d'une spatule. Dans un premier temps, la spatule est plongée dans la phase PG. Lorsque celle-ci est retirée, on observe l'aspect de la phase PG qui s'écoule de la spatule et on note la fréquence d'apparition de microgels (ou inclusions gélifiées) dans l'écoulement.

Plus particulièrement et dans tous les essais qui suivent, la fréquence d'apparition des microgels est quantifiée de la manière suivante :
- « Présence » de microgels : >20 microgels/g de polyglycérol,
- « Faible présence » de microgels : 5-20 microgels/g de polyglycérol,
- « Très faible présence » de microgels : <5 microgels/g de polyglycérol. Analyse IR du PGE :
   3360 cm⁻¹ (√OH) ; 2940 cm⁻¹ (√CH saturé) ; 2832 cm⁻¹ (√CH saturé) ; 1735 cm⁻¹ (√C=O); 1450 et 1372 cm⁻¹ (∂CH saturé) ; 1108 cm⁻¹ (∂C=O ester) ; 1040 cm⁻¹ (∂C-O alcool).

Analyse IR du PG : Absence de bande caractéristique de fonction ester.

Le PGE issu de ce procédé est utilisé comme agent compatibilisant 1 dans l'Exemple 3 ci-après.

### Exemple 2 : Effets du mode d'addition de l'acide gras sur le procédé de l'invention

### Essai 2 : Addition en masse

Dans un réacteur en verre de 500 ml équipé d'une agitation mécanique, d'un système de distillation et d'un système de barbotage d'azote, on introduit 286,4 g (2,43 mole) de carbonate de glycérol, 173,2 g (0,61 mole) d'Iso C18:0 Radiacid® 909 de la société OLEON et 1,19 g (0,03 mole) de catalyseur, du NaOH solide (soit 5 % molaire par rapport à l'acide isostéarique). Le milieu réactionnel est chauffé pendant 5 heures à 200°C. Après refroidissement, on obtient 322,5 g de masse réactionnelle dont 229 g de PGE en phase supérieure d'indice d'acide 0,3 mg KOH/g et 93,6 g de PG en phase inférieure, soit un ratio PGE/PG de 71/29. La quantité d'eau distillée est de 7,2 g.

### Essai 3 : Addition en 3 fractions

Dans un réacteur en verre de 500 ml équipé d'une agitation mécanique, d'un système de distillation et d'un système de barbotage d'azote, on introduit 312,9 g de carbonate de glycérol et 1,32 g de NaOH solide. La température du milieu réactionnel est portée graduellement à 180°C. A cette température, un premier ajout de 49,28 g d'Iso C18:0 Radiacid® 909 de la société OLEON est réalisé puis la température du milieu réactionnel est portée à 190°C. Une heure après le premier ajout d'Iso C18:0, un second ajout de 92,8 g d'Iso C18:0 est réalisé. Une demi-heure après le deuxième ajout, un dernier ajout de 47,5 g d'Iso C18:0 est réalisé. En fin d'ajout, la température du milieu réactionnel est portée à 200°C. La réaction est maintenue 5 heures à 200°C. Après refroidissement, on obtient 370 g de masse réactionnelle dont 311,8 g de PGE en phase supérieure d'indice d'acide : 0,2 mg KOH/g et 58,7 g de PG en phase inférieure, soit un ratio PGE/PG de 84/16. La quantité d'eau distillée est de 3,9 g.

### Essai 4 : Addition goutte à goutte

Dans un réacteur en verre de 500 ml équipé d'une agitation mécanique, d'un système de distillation et d'un système de barbotage d'azote, on charge dans le réacteur 377,28 g de carbonate de glycérol et 1,58 g de NaOH solide. La température du milieu réactionnel est portée graduellement à 180°C. 221,28 g d'Iso C18:0 Radiacid® 909 de la société OLEON sont additionnés goutte à goutte pendant 1 heure au moyen d'une ampoule de coulée. La réaction est maintenue pendant 5 heures à 180°C. Après refroidissement, on obtient 361 g de masse réactionnelle dont 323 g de PGE en phase supérieure d'indice d'acide : 0,2 mg KOH/g et 37,8 g de PG en phase inférieure, soit un ratio PGE/PG de 90/10. La quantité d'eau distillée est de 3,04 g.

Les trois essais réalisés dans l'exemple 2 sont portés dans le tableau 1 suivant :

**Tableau 1 : Récapitulatif des essais 2 à 4 réalisés dans l'exemple 2**

| **Essai** | **Acide gras** | **Température (°C)** | **Mode et temps d'introduction de l'acide gras** | **Ratio massique PGE/PG** | **Indice d'acide final (mgKOH/g)** |
|---|---|---|---|---|---|
| 2 | Iso C18:0 | 200 | En masse, 0 | 71/29 | 0,3 |
| 3 | Iso C18:0 | 190-200 | En 3 fois, sur 1h30 | 84/16 | 0,2 |
| 4 | Iso C18:0 | 180 | Goutte à goutte pendant 1h | 90/10 | 0,2 |

### Exemple 3 : Procédés de préparation d'agents compatibilisants

### Essai 5 : Préparation de l'agent compatibilisant 2

Dans un réacteur en verre de 500 ml équipé d'une agitation mécanique, d'une sortie directe de distillation et d'une introduction d'azote dans le milieu réactionnel, on charge 495,6 g (4,20 mole) de carbonate de glycérol et 3,9 g (0,05 mole) d'hydroxyde de calcium (chaux). Le milieu réactionnel est ensuite chauffé progressivement à 210°C en présence de l'agent compatibilisant 1 chargé à 1 % en poids par rapport à la masse totale réactionnelle (carbonate de glycérol + chaux + acide isostéarique), puis 298,5 g (1,05 mole) d'Iso C18:0 Radiacid® 909 de la société OLEON sont additionnés goutte à goutte au moyen d'une pompe péristaltique pendant 3 heures. Un prélèvement est réalisé pour titrage de l'indice d'acide en fin d'introduction puis le milieu réactionnel est maintenu pendant 3 heures à 210°C. L'évolution de la réaction est suivie par titrage d'indice d'acide sur prélèvements. Après décantation à 90°C, le ratio PGE/PG obtenu est de 85/15.

La phase PGE obtenue est utilisée en tant qu'agent compatibilisant 2.

Ce procédé de préparation de l'agent compatibilisant 2 est également un procédé de production d'ester(s) de polyglycérol conforme à l'invention.

### Essai 6 : Préparation de l'agent compatibilisant 3

Dans un réacteur en verre de 500 ml équipé d'une agitation mécanique, d'une sortie directe de distillation et d'une introduction d'azote dans le milieu réactionnel, on charge 495,6 g (4,20 mole) de carbonate de glycérol que l'on chauffe progressivement à 160°C. On charge ensuite l'agent compatibilisant 2 à 1 % en poids par rapport à la masse totale réactionnelle (carbonate de glycérol + chaux + acide isostéarique) puis 3,9 g (0,05 mole) d'hydroxyde de calcium (chaux). Le milieu réactionnel est rapidement chauffé à 210°C. 298,5 g (1,05 mole) d'Iso C18:0 Radiacid® 909 de la société OLEON sont alors additionnés goutte à goutte au moyen d'une pompe péristaltique pendant 3 heures. Un prélèvement est réalisé pour titrage de l'indice d'acide en fin d'introduction puis le milieu réactionnel est maintenu pendant 3 heures à 210°C. L'évolution de la réaction est suivie par titrage d'indice d'acide sur prélèvements. Un rapport massique PGE/PG de 94/6 est obtenu après décantation à 90°C. La phase PGE a un indice d'acide de 1,9 mg KOH/g. La phase PG contient peu de microgels.

Le PGE obtenu sert d'agent compatibilisant 3 dans les synthèses de l'Exemple 4.

Ce procédé de préparation de l'agent compatibilisant 3 est également un procédé de production d'ester(s) de polyglycérol conforme à l'invention.

### Exemple 4: Procédés de production d'ester(s) de Polyglycérol en présence d'un agent compatibilisant

### Essai 7

Dans un réacteur en verre de 500 ml équipé d'une agitation mécanique, d'une sortie directe de distillation et d'une introduction d'azote dans le milieu réactionnel, on charge 495,6 g (4,20 mole) de carbonate de glycérol que l'on chauffe progressivement à 160°C. On charge ensuite l'agent compatibilisant 3 à 1 % en poids par rapport à la masse totale réactionnelle (carbonate de glycérol + chaux + acide isostéarique) puis 0,4 g (0,005 mole) d'hydroxyde de calcium (chaux). Le milieu réactionnel est rapidement chauffé à 210°C. 298,5 g (1,05 mole) d'Iso C18:0 Radiacid® 909 de la société OLEON sont alors additionnés goutte à goutte au moyen d'une pompe péristaltique pendant 3 heures. Un prélèvement est réalisé pour titrage de l'indice d'acide en fin d'introduction puis le milieu réactionnel est maintenu pendant 3 heures à 210°C. L'évolution de la réaction est suivie par titrage d'indice d'acide sur prélèvements. Un rapport massique PGE/PG de 96/4 est obtenu après décantation à 90°C. La phase PGE de coloration Gardner 5,5, a un indice d'acide de 1,9 mg KOH/g. La phase PG contient très peu de microgels. La phase PGE est dispersable à 1 % en poids dans de l'eau déminéralisée après agitation à température ambiante. La dispersion stockée à température ambiante est stable dans le temps.

La dispersion est préparée par agitation manuelle forte d'un flacon de verre de 125 cm³ contenant 1 g de PGE et 100 ml d'eau déminéralisée. L'agitation est arrêtée lorsque le milieu semble homogène par observation visuelle. Le maintien ou non de l'aspect homogène au cours du stockage à température ambiante est par la suite vérifiée sur une période d'au moins 5 jours. Un aspect hétérogène se traduit par une décantation de phase organique en surface, un dépôt en fond de flacon, un dépôt sur les parois du verre et/ou des inclusions de phase organique.

### Essai 8

Dans un réacteur en verre de 500 ml équipé d'une agitation mécanique, d'une sortie directe de distillation et d'une introduction d'azote dans le milieu réactionnel, on charge 495,6 g (4,20 mole) de carbonate de glycérol que l'on chauffe progressivement à 160°C. On charge ensuite l'agent compatibilisant 3 à 1 % en poids par rapport à la masse totale réactionnelle (carbonate de glycérol + chaux + acide isostéarique) puis 0,4 g (0,005 mole) d'hydroxyde de calcium (chaux). Le milieu réactionnel est rapidement chauffé à 210°C. 298,5 g (1,05 mole) d'Iso C18:0 Radiacid® 909 de la société OLEON sont alors additionnés goutte à goutte au moyen d'une pompe péristaltique pendant 3 heures. Un prélèvement est réalisé pour titrage de l'indice d'acide en fin d'introduction puis le milieu réactionnel est maintenu pendant 7 heures à 210°C. L'évolution de la réaction est suivie par titrage d'indice d'acide sur prélèvements. Un rapport massique PGE/PG de 96/4 est obtenu après décantation à 90°C. La phase PGE de coloration Gardner 5, a un indice d'acide de 0,3 mg KOH/g. La phase PG contient très peu de microgels. La phase PGE est dispersable à 1 % en poids dans de l'eau déminéralisée après agitation à température ambiante. La dispersion stockée à température ambiante est stable dans le temps.

La Figure 1 montre le chromatogramme d'exclusion stérique du produit PGE obtenu en fin de réaction, après décantation.

Pour réaliser ce chromatogramme, 0,3 g d'échantillon de PGE sont dilués avec 5 g de tétrahydrofurane (THF) 100 % qui est l'éluent utilisé pour l'analyse.

Une aliquote est analysée selon les conditions suivantes :
Système GPC WATERS 717 Plus
Pompe binaire WATERS 1525, isocratique
Détecteur RI WATERS 2414
Logiciel d'acquisition d'analyse : Breeze v3.30
Colonne : PLgel (Agilent Technologies) 300 x 7,5 mm ; 5 mm (50 Å, 50 Å, 100 Å, 500 Å, 1000 Å) soit deux colonnes de porosité 50 Å et une colonne pour chacune des autres porosités.
Volume d'injection : 10 ml
Température de four de colonne : 35°C
Débit : 1 ml/min
Température du détecteur : 35°C
Sensibilité du détecteur : 4

### Exemple 5 : Procédé de production d'ester(s) de Polyglycérol par réaction en masse

### Essai 9

Dans un réacteur en verre de 500 ml équipé d'une agitation mécanique, d'une sortie directe de distillation et d'une introduction d'azote dans le milieu réactionnel, on charge 495,6 g (4,20 mole) de carbonate de glycérol, l'agent compatibilisant 3 à 1 % en poids par rapport à la masse totale réactionnelle (carbonate de glycérol + chaux + acide isostéarique), 0,4 g (0,005 mole) d'hydroxyde de calcium (chaux) et 298,5 g (1,05 mole) d'Iso C18:0 Radiacid® 909 de la société OLEON. Un prélèvement est réalisé pour titrage de l'indice d'acide en fin d'introduction puis le milieu réactionnel est chauffé à 210°C pendant 4 heures. L'évolution de la réaction est suivie par titrage d'indice d'acide sur prélèvements. Après décantation à 90°C, un rapport massique de 80/20 est obtenu. L'indice d'acide de la phase PGE obtenue est de 0,2 mg KOH/g La phase PG contient peu de microgels. La phase PGE n'est pas dispersable à 1 % en poids dans l'eau déminéralisée.

### Exemple 6 : Procédé de production d'ester(s) de polyglycérol sans agent compatibilisant

### Essai 10

Dans un réacteur en verre de 500 ml équipé d'une agitation mécanique, d'une sortie directe de distillation et d'une introduction d'azote dans le milieu réactionnel, on charge 495,6 g (4,20 mole) de carbonate de glycérol que l'on chauffe progressivement à 160°C. On charge ensuite 0,4 g (0,005 mole) d'hydroxyde de calcium (chaux). Le milieu réactionnel est rapidement chauffé à 210°C. 298,5 g (1,05 mole) d'Iso C18:0 Radiacid® 909 de la société OLEON sont alors additionnés goutte à goutte au moyen d'une pompe péristaltique pendant 3 heures. Un prélèvement est réalisé pour titrage de l'indice d'acide en fin d'introduction puis le milieu réactionnel est maintenu pendant 7 heures à 210°C. L'évolution de la réaction est suivie par titrage d'indice d'acide sur prélèvements. Un rapport massique PGE/PG de 81/19 est obtenu après décantation à 90°C. La phase PGE a un indice d'acide de 0,8 mg KOH/g. La phase PG contient peu de microgels. La phase PGE n'est pas dispersable à 1 % en poids dans l'eau déminéralisée.

### Exemple 7 : Procédé de production d'ester(s) de Polyglycérol par ajout en goutte à goutte de carbonate de glycérol

### Essai 11

Dans un réacteur verre de 500 ml équipé d'une agitation mécanique, d'une sortie directe de distillation et d'une introduction d'azote dans le milieu réactionnel, on charge 200 g (0,70 mole) d'Iso C18:0 Radiacid® 909 de la société OLEON. L'Iso C18:0 est chauffé à 160°C. Lorsque cette température est atteinte, 0,26 g (0,0035 mole) d'hydroxyde de calcium et 5,18 g d'agent compatibilisant 2 sont ajoutés. Ensuite, le milieu réactionnel est rapidement chauffé à 210°C avant de démarrer l'addition goutte à goutte de 333,1 g (2,82 mole) de carbonate de glycérol pendant 3 heures au moyen d'une pompe péristaltique. On observe un moussage important difficilement contrôlable dans le milieu réactionnel pendant la première heure d'addition. Un prélèvement est réalisé pour titrage de l'indice d'acide en fin d'introduction puis le milieu réactionnel est maintenu pendant 1 heure 30 supplémentaire à 210°C. Après refroidissement, on obtient 359 g de masse réactionnelle dont 290,6 g de PGE d'indice d'acide : 0,2 mg KOH/g en phase supérieure et 68 g de PG en phase inférieure, après décantation ; soit un ratio PGE/PG de 81/19. La quantité d'eau distillée est de 18,2 g.

Une dispersion à 1 % en poids dans l'eau déminéralisée n'est pas stable après un jour de stockage. On observe la formation de dépôt après un jour.

L'ensemble des procédés de production de PGE mettant en oeuvre l'hydroxyde de calcium comme catalyseur est synthétisé dans le tableau 2 (tableaux 2a et 2b) ci-après.

### Exemple 8 : Procédé de production d'ester(s) de Polyglycérol avec un ratio carbonate de glycérol/acide gras de 6/1

### Essai 12

Dans un réacteur en verre de 1000 ml équipé d'une agitation mécanique, d'une sortie directe de distillation et d'une introduction d'azote dans le milieu réactionnel, on charge 498,2 g (4,22 mole) de carbonate de glycérol et 1,22 g (0,015 mole) d'hydroxyde de calcium (chaux). Le milieu réactionnel est chauffé à 210°C, puis 200,86 g (0,706 mole) d'acide isostéarique Radiacid® 909 de la société OLEON sont additionnés goutte à goutte au moyen d'une pompe péristaltique pendant 3h30. Un prélèvement est réalisé pour titrage de l'indice d'acide en fin d'introduction puis le milieu réactionnel est maintenu pendant 3 heures à 210°C. L'évolution de la réaction est suivie par titrage d'indice d'acide sur prélèvements. Après décantation à 90°C, on obtient 300 g d'ester de polyglycérol (PGE) d'indice d'acide : 0,3 mg KOH/g en phase supérieure et 122,77 g de polyglycérol (PG) en phase inférieure contenant des microgels ; soit un ratio PGE/PG de 71/29. 24 g d'eau distillée sont récupérés.

La phase PGE est dispersable à 1 % en poids dans de l'eau déminéralisée après agitation à température ambiante. La dispersion stockée à température ambiante reste stable dans le temps.
Analyse IR du PG : Absence de bande caractéristique de fonction ester.

### Essai 13

Dans un réacteur en verre de 500 ml équipé d'une agitation mécanique, d'une sortie directe de distillation et d'une introduction d'azote dans le milieu réactionnel, on charge 324,50 g (2,75 mole) de carbonate de glycérol et 1,71 g (0,02 mole) d'hydroxyde de calcium (chaux). Le milieu réactionnel est chauffé à 210°C, puis 131,20 g (0,46 mole) d'acide isostéarique Radiacid® 909 de la société OLEON sont additionnés goutte à goutte au moyen d'une pompe péristaltique pendant 3 heures. Un prélèvement est réalisé pour titrage de l'indice d'acide en fin d'introduction puis le milieu réactionnel est maintenu pendant 3 heures à 210°C. L'évolution de la réaction est suivie par titrage d'indice d'acide sur prélèvements. Après décantation à 90°C, on obtient 189,9 g d'ester de polyglycérol (PGE) d'indice d'acide: 0,64 mg KOH/g en phase supérieure et 78,34 g de polyglycérol (PG) en phase inférieure contenant des microgels ; soit un ratio PGE/PG de 71/29. 19,8 g d'eau distillée sont récupérés.

La phase PGE est dispersable à 1 % en poids dans de l'eau déminéralisée après agitation à température ambiante. La dispersion stockée à température ambiante reste stable dans le temps.
Analyse IR du PG : Absence de bande caractéristique de fonction ester.

### Essai 14

Dans un réacteur en verre de 500 ml équipé d'une agitation mécanique, d'une sortie directe de distillation et d'une introduction d'azote dans le milieu réactionnel, on charge 500,7 g (4,24 mole) de carbonate de glycérol que l'on chauffe progressivement à 160°C. On charge ensuite en tant qu'agent compatibilisant 6,6 g de PGE issu de l'essai 12 (1 % pds par rapport à la masse réactionnelle totale (carbonate de glycérol + chaux + acide isostéarique) puis 0,3 g (0,0041 mole) d'hydroxyde de calcium (chaux). Le milieu réactionnel est rapidement chauffé à 210°C. 200 g (0,70 mole) d'acide isostéarique (désigné ci-après « Iso C18 :0 ») Radiacid® 909 de la société OLEON sont alors additionnés goutte à goutte au moyen d'une pompe péristaltique pendant 3 heures. Un prélèvement est réalisé pour titrage de l'indice d'acide en fin d'introduction puis le milieu réactionnel est maintenu pendant 4 heures à 210°C. L'évolution de la réaction est suivie par titrage d'indice d'acide sur prélèvements. Un rapport massique PGE/PG de 82/18 est obtenu après décantation à 90°C. La phase PGE a un indice d'acide de 0,2 mg KOH/g. La phase PG contient très peu de microgels. 22 g d'eau distillée sont récupérés.

La phase PGE est dispersable à 1 % en poids dans de l'eau déminéralisée après agitation à température ambiante. La dispersion stockée à température ambiante reste stable dans le temps.

### Essai 15

Dans un réacteur en verre de 500 ml équipé d'une agitation mécanique, d'une sortie directe de distillation et d'une introduction d'azote dans le milieu réactionnel, on charge 284 g (2,40 mole) de carbonate de glycérol que l'on chauffe progressivement à 160°C. On charge ensuite 0,3 g (0,0041 mole) d'hydroxyde de calcium (chaux). Le milieu réactionnel est rapidement chauffé à 210°C. 113,7 g (0,40 mole) d'acide isostéarique (désigné ci-après « Iso C18 :0 ») Radiacid® 909 de la société OLEON sont alors additionnés goutte à goutte au moyen d'une pompe péristaltique pendant 3 heures. Un prélèvement est réalisé pour titrage de l'indice d'acide en fin d'introduction puis le milieu réactionnel est maintenu pendant 3 heures à 210°C. L'évolution de la réaction est suivie par titrage d'indice d'acide sur prélèvements. Un rapport massique PGE/PG de 74/26 est obtenu après décantation à 90°C. La phase PGE a un indice d'acide de 0,2 mg KOH/g. La phase PG contient peu de microgels. 20 g d'eau distillée sont récupérés.

La phase PGE n'est pas dispersable à 1 % en poids dans de l'eau déminéralisée après agitation à température ambiante.

### Exemple 9: Procédé de production d'ester(s) de polyglycérol par estérification directe d'un Polyglycérol

### Essai 16

Cet essai est réalisé avec du polyglycérol-4, afin de pouvoir être comparé aux essais réalisés avec un ratio molaire carbonate de glycérol/acide gras de 4/1.

Dans un réacteur en verre de 500 ml équipé d'une agitation mécanique, d'une sortie directe de distillation et d'une introduction d'azote dans le milieu réactionnel, on charge 311,25 g de polyglycérol-4 (commercialisé par Solvay) que l'on chauffe progressivement à 160°C. On charge ensuite l'agent compatibilisant 3 à 1 % en poids par rapport à la masse totale réactionnelle (polyglycérol-4 + chaux + acide isostéarique) puis 0,4 g (0,005 mole) d'hydroxyde de calcium (chaux). Le milieu réactionnel est rapidement chauffé à 210°C. Une fois cette température atteinte, 298,5 g d'Iso C18:0 Radiacid® 909 de la société OLEON (ratio massique PG-4/acide isostéarique = 1,10/1) sont alors additionnés goutte à goutte au moyen d'une pompe péristaltique pendant 3 heures. Un prélèvement est réalisé pour titrage de l'indice d'acide en fin d'introduction puis le milieu réactionnel est maintenu pendant 3 heures à 210°C. L'évolution de la réaction est suivie par titrage d'indice d'acide sur prélèvements. Un rapport massique PGE/PG de 72/28 est obtenu après décantation à 90°C. La phase PGE a un indice d'acide de 3,2 mg KOH/g. La phase inférieure, contenant le PG, contient des microgels. La phase PGE n'est pas dispersable dans de l'eau déminéralisée après agitation à température ambiante.

### Exemple 10: Procédé de production d'ester(s) de Polyglycérol en l'absence de catalyseur

### Essai 17

Dans un réacteur en verre de 500 ml équipé d'une agitation mécanique, d'une sortie directe de distillation et d'une introduction d'azote dans le milieu réactionnel, on charge 283,4 g (2,40 mole) de carbonate de glycérol que l'on chauffe progressivement à 160°C. On charge ensuite en tant qu'agent compatibilisant 6,6 g de PGE issu de l'essai 8 (1 % pds par rapport à la masse réactionnelle totale (carbonate de glycérol + chaux + acide isostéarique). Le milieu réactionnel est rapidement chauffé à 210°C. 170,7 g (0,60 mole) d'acide isostéarique (désigné ci-après « Iso C18:0») Radiacid®909 de la société OLEON sont alors additionnés goutte à goutte au moyen d'une pompe péristaltique pendant 3 heures. Un prélèvement est réalisé pour titrage de l'indice d'acide en fin d'introduction puis le milieu réactionnel est maintenu pendant 3 heures à 210°C. L'évolution de la réaction est suivie par titrage d'indice d'acide sur prélèvements. Un rapport massique PGE/PG de 85/15 est obtenu après décantation à 90°C. La phase PGE a un indice d'acide de 0,2 mg KOH/g. 13,18 g d'eau distillée sont récupérées. La phase PGE n'est pas dispersable à 1 % en poids dans de l'eau déminéralisée après agitation à température ambiante.

### Exemple 11 : Procédé de production d'ester(s) de Polyglycérol avec un ratio carbonate de glycéro/lacide gras de 10/1

### Essai 18

Dans un réacteur en verre de 500 ml équipé d'une agitation mécanique, d'une sortie directe de distillation et d'une introduction d'azote dans le milieu réactionnel, on charge 295,2 g (2,50 mole) de carbonate de glycérol, 0,095 g (0,0013 mole) d'hydroxyde de calcium (chaux) et 3,6 g de PGE issu de l'essai 21 en tant qu'agent compatibilisant que l'on chauffe en 30 minutes à 210°C. A 210°C, 71,2 g (0,25 mole) d'acide isostéarique (désigné ci-après « Iso C18 :0 ») Radiacid®909 de la société OLEON sont alors additionnés goutte à goutte au moyen d'une pompe péristaltique pendant 3 heures. Un prélèvement est réalisé pour titrage de l'indice d'acide en fin d'introduction puis le milieu réactionnel est maintenu pendant 2h30 à 210°C. L'évolution de la réaction est suivie par titrage d'indice d'acide sur prélèvements. Un rapport massique PGE/PG de 60/40 est obtenu après décantation à 90°C avec 143,85 g de phase PGE d'un indice d'acide : 0,2 mg KOH/g et 95,86 g de phase PG. 15,2 g d'eau distillée sont récupérées.

La phase PGE est dispersable à 1 % en poids dans de l'eau déminéralisée après agitation à température ambiante. La dispersion stockée à température ambiante reste stable dans le temps.

**Tableau 2a : Récapitulatif des essais avec un ratio 4/1 des Exemples 1, 3 à 7, 9 et 10**

| **Essai** | **Réactif / Mode et temps d'introduction** | **Ratio molaire carbonate de glycérol/ acide gras** | **Agent compati--bilisant (%*)** | **Catalyseur Ca(OH)₂ (% molaire /acide gras)** | **Température (°C) / temps de réaction (h)** | **Ratio massique final PGE/PG** | **Indice d'acide PGE (mg KOH/g)** | **Microgels** | **Stabilité**** |
|---|---|---|---|---|---|---|---|---|---|
| 1 | Iso C18:0 GAG***, 3 h | 4/1 | - | Ca(OH)₂ 4,8 | 200 / 3h puis 210 / 3h | 92/8 | 2,3 | Présence | Dépôt après 5 jours |
| 10 | Iso C18:0 GAG***, 3 h | 4/1 | - | Ca(OH)₂ 0,5 | 210 / 10h | 81/19 | 0,8 | Faible présence | Non dispersable |
| 17 | Iso C18:0 GAG***, 3h | 4/1 | 1 | - | 210 / 6h | 85/15 | 0,2 | Faible Présence | Non dispersable |
| 5 | Iso C18:0 GAG***, 3 h | 4/1 | 1 | Ca(OH)₂ 4,8 | 210 / 6h | 85/15 | 1,9 | Faible Présence | OK |
| 6 | Iso C18:0 GAG***, 3 h | 4/1 | 1 | Ca(OH)₂ 4,8 | 210 / 6h | 94/6 | 1,9 | Faible Présence | OK |
| 7 | Iso C18:0 GAG***, 3 h | 4/1 | 1 | Ca(OH)₂ 0,5 | 210 / 6h | 96/4 | 1,9 | Très Faible Présence | OK |
| 8 | Iso C18:0 GAG***, 3 h | 4/1 | 1 | Ca(OH)₂ 0,5 | 210 / 10h | 96/4 | 0,3 | Très faible présence | OK |
| 11 | Carbonate de glycérol GAG***, 3 h | 4/1 | 1 | Ca(OH)₂ 0,5 | 210 / 4h30 | 81/19 | 0,2 | Faible présence | Dépôt après 1 jour |
| 9 | Masse | 4/1 | 1 | Ca(OH)₂ 0,5 | 210 / 4h | 80/20 | 0,2 | Faible présence | Non dispersable |
| 16 | Iso C18:0 GAG***, 3 h | Polyglycérol, 1,1/1 | 1 | Ca(OH)₂ 0,5 | 210 / 6h | 72/28 | 3,2 | Présence | Non dispersable |

| | | | | | | | | | |
|---|---|---|---|---|---|---|---|---|---|
| * % en poids par rapport à la masse totale réactionnelle (carbonate de glycérol + hydroxyde de calcium + acide isostéarique) ** Stabilité de la dispersion à température ambiante à 1% dans de l'eau déminéralisée *** GAG : goutte à goutte | | | | | | | | | |

**Tableau 2b : Récapitulatif des essais des Exemples 8 et 11**

| **Essai** | **Réactif / Mode et temps d'introduction** | **Ratio molaire carbonate de glycérol/acid e gras** | **Agent compatib ilisant (%*)** | **Catalyseur Ca(OH)₂ (% molaire /acide gras)** | **Température (°C) / temps de réaction (h)** | **Ratio massique final PGE/PG** | **Indice d'acide PGE (mg KOH/g)** | **Microgels** | **Stabilité**** |
|---|---|---|---|---|---|---|---|---|---|
| 12 | Iso C18:0 GAG***, 3h30 | 6/1 | - | Ca(OH)₂ 2,1 | 210 / 6h30 | 71/29 | 0,3 | Présence | OK |
| 13 | Iso C18:0 GAG***, 3h | 6/1 | - | Ca(OH)₂ 4,3 | 210 / 6h | 71/29 | 0,6 | Présence | OK |
| 15 | Iso C18:0 GAG***, 3h | 6/1 | - | Ca(OH)₂ 1,0 | 210 / 6h | 74/26 | 0,2 | Faible présence | Non dispersable |
| 14 | Iso C18:0 GAG***, 3h | 6/1 | 1 | Ca(OH)₂ 0,6 | 210 / 7h | 82/18 | 0,2 | Très faible présence | OK |
| 18 | Iso C18:0 GAG***, 3h | 10/1 | 1 | Ca(OH)₂ 0,5 | 210 / 6h | 60/40 | 0,2 | Faible Présence | OK |

| | | | | | | | | | |
|---|---|---|---|---|---|---|---|---|---|
| * % en poids par rapport à la masse totale réactionnelle (carbonate de glycérol + hydroxyde de calcium + acide isostéarique) ** Stabilité de la dispersion à température ambiante à 1% dans de l'eau déminéralisée *** GAG : goutte à goutte | | | | | | | | | |

## Revendications

1. Procédé de production d'un ester de polyglycérol comprenant la mise en contact, dans un réacteur, d'un acide gras et de carbonate de glycérol dans lequel le carbonate de glycérol est placé dans le réacteur, et au moins une partie de l'acide gras est ajouté dans le réacteur contenant le carbonate de glycérol chauffé à une température supérieure ou égale à 150°C.

2. Procédé selon la revendication 1, dans lequel la quantité totale d'acide gras est ajoutée dans le réacteur en une fois.

3. Procédé selon la revendication 1, dans lequel la quantité totale d'acide gras est ajoutée dans le réacteur en au moins deux fois.

4. Procédé selon la revendication 3, dans lequel au moins une partie de l'acide gras est ajoutée goutte à goutte dans le réacteur.

5. Procédé selon l'un quelconque des revendications 1 à 4, dans lequel la quantité totale d'acide gras est ajoutée dans le réacteur sur une période de temps d'au moins 30 min.

6. Procédé selon l'une quelconque des revendications 1 à 5, dans lequel un catalyseur basique est introduit dans le réacteur.

7. Procédé selon la revendication 6, dans lequel le catalyseur basique est l'hydroxyde de calcium et est introduit à une quantité de 0,1 à 7,5% molaire par rapport à la quantité totale d'acide gras introduite.

8. Procédé selon l'une quelconque des revendications 1 à 7, dans lequel un agent compatibilisant est introduit dans le réacteur.

9. Procédé selon la revendication 8, dans lequel l'agent compatibilisant est un ester de polyglycérol.

10. Procédé selon l'une quelconque des revendications 1 à 9, dans lequel le début de l'ajout d'au moins une partie de l'acide gras dans le réacteur est effectué dans un délai compris entre le moment où le carbonate de glycérol atteint une température d'au moins 150°C, et jusqu'à 2h00 après que cette température soit atteinte.

11. Procédé selon l'une quelconque des revendications 1 à 10, dans lequel le carbonate de glycérol et l'acide gras sont chauffés à une température comprise entre 170°C et 250°C.

12. Procédé selon l'une quelconque des revendications 1 à 11, dans lequel le ratio molaire des quantités totales de carbonate de glycérol/acide gras qui sont introduits dans le réacteur, est supérieur ou égal à 2.

13. Procédé selon l'une quelconque des revendications précédentes, comportant une étape de décantation à l'issue de la réaction, suivie d'une étape de séparation de l'ester de polyglycérol.

14. Produit susceptible d'être obtenu par le procédé selon l'une quelconque des revendications 1 à 13.

15. Utilisation du produit selon la revendication 14, comme émulsifiant.
